# EUROPEAN PATENT SPECIFICATION

(11) **EP 2 688 948 B1**
(45) Date of publication and mention of the grant of the patent: **14.01.2015**
(21) Application number: 12710080.8
(22) Date of filing: 21.03.2012
(51) Int. Cl.: C08K 5/00, C08F 299/04, C08K 3/00

(54) **PROCESS FOR THE PREPARATION OF AN ACCELERATOR SOLUTION**
VERFAHREN ZUR HERSTELLUNG EINER BESCHLEUNIGERLÖSUNG
PROCÉDÉ DE PRÉPARATION D'UNE SOLUTION ACCÉLÉRATRICE

(30) Priority: 24.03.2011 EP 11159564; 25.03.2011 US 201161467569 P
(43) Date of publication of application: 29.01.2014
(73) Proprietor: Akzo Nobel Chemicals International B.V., 3811 MH Amersfoort (NL)
(72) Inventor: REIJNDERS, Johannes Martinus Gerardus Maria, NL-8162 ED Epe (NL); KOERS, Frederik Willem Karel, NL-7214 BZ Epse (NL); TALMA, Auke Gerardus, NL-7437 PM Bathmen (NL)
(74) Representative: Akzo Nobel IP Department
(86) International application number: PCT/EP2012/054933
(87) International publication number: WO 2012/126919

(56) References cited:
- WO-A1-2006/090911
- WO-A1-2006/128816
- WO-A1-2008/119783

## Description

The present invention relates to a process for the preparation of an accelerator solution suitable for forming a redox system with peroxides.

Redox systems can be applied for resin curing. Conventional redox systems comprise an oxidizing agent (e.g. a peroxide) and a soluble transition metal ion as accelerator. The accelerator serves to increase the activity of the oxidizing agent at lower temperatures and, consequently, to speed up the curing rate.

Accelerator systems can be added to the resin to be cured in different ways. One method involves the addition of the individual accelerator ingredients to the resin, before the peroxide is added. This can be done just in advance of peroxide addition or days or weeks before that. In the latter case, we refer to a pre-accelerated resin composition, which comprises the resin and the accelerator ingredients and can be stored until further use and cure with the peroxide. Another method involves the pre-preparation of an accelerator solution containing the accelerator ingredients, which solution can be stored until further use and addition to the resin. A pre-accelerated resin can be prepared by either adding the individual ingredients of the accelerator system to the resin or by adding these ingredients in admixture in the form of an accelerator solution.

It has now been found that the performance of an accelerator solution is influenced by its manner of preparation.

The invention relates to a process for the preparation of an accelerator solution suitable for forming a redox system with peroxides, comprising the step of adding a transition metal salt or complex to a liquid formulation comprising a hydroxy-functional solvent and a nitrogen-containing base at a temperature in the range 50-200°C.

This process allows for the preparation of an accelerator solution that is storage stable, meaning that no sedimentation is observed when stored for six months at ambient temperature. Furthermore, this process leads to reduced production time of the solution, because of the quick dissolution of Cu.

The invention also relates to a process for pre-accelerating a curable resin by mixing an accelerator solution obtainable by the above process into a curable resin.

According to the process of the present invention, a liquid formulation comprising a hydroxy-functional solvent and a nitrogen-containing base is heated to a temperature in the range 50-200°C, preferably 80-110°C. Alternatively, the nitrogen-containing base is added to a hydroxyl-functional solvent-containing solution already having a temperature in that range. Or *vice versa.*

At this temperature, the transition metal salt or complex is added to the mixture and allowed to dissolve.

During the process, the formulation is preferably stirred. The resulting clear solution is then cooled down to room temperature. The process takes about 1 to 8 hours.

Preferred transition metals for use in the process according to the present invention are transition metals other than Co. More preferably, the transition metal is selected from the group consisting of Mn, Fe, Cu, and V. It is also possible to use two or more transition metals. Preferably, at least one of the transition metals is selected from Mn, Fe, and Cu.

Suitable compounds of the first and second transition metals are salts and complexes thereof, such as their halides, nitrate, sulphate, sulphonate, phosphate, phosphonate, oxide, or carboxylates. Examples of suitable carboxylates are lactate, 2-ethyl hexanoate, acetate, proprionate, butyrate, oxalate, laurate, oleate, linoleate, palmitate, stearate, acetyl acetonate, octanoate, nonanoate, heptanoate, neodecanoate, and naphthenate.

Preferred manganese compounds are manganese chloride, nitrate, sulphate, lactate, 2-ethyl hexanoate, octanoate, nonanoate, heptanoate, neodecanoate, naphthenate, and acetate and the Mn complexes of pyridine, bipyridine and derivatives thereof, and of the tridentate, tetradentate, pentadentate, or hexadentate nitrogen donor ligands disclosed in WO 2011/83309. Any one of Mn(II), Mn(III), Mn(IV) and Mn(VII) compounds can be used.

Preferred copper compounds are copper chloride, nitrate, sulphate, lactate, 2-ethyl hexanoate, octanoate, nonanoate, heptanoate, neodecanoate, naphthenate, and acetate. Both Cu(I) and Cu(II) salts can be used.

Preferred iron compounds are iron chloride, nitrate, sulphate, lactate, 2-ethyl hexanoate, octanoate, nonanoate, heptanoate, neodecanoate, naphthenate, acetate, and iron complexes of pyridine, bipyridine and derivatives thereof, and the tridentate, tetradentate, pentadentate, or hexadentate nitrogen donor ligands of WO 2011/83309.. Both Fe(II) and Fe(III) can be used. More preferably, it is an iron(II) or iron (III) complex of a pentadentate nitrogen donor ligand as further disclosed in WO 2011/83309.

Preferred nitrogen donor ligands according to WO 2011/83309, for both Mn and Fe, are the bispidon ligands and the TACN-Nx ligands. The preferred bispidon ligand is dimethyl-2,4-di-(2-pyridyl)-3-methyl-7-(pyridin-2-ylmethyl)-3,7-diazabicyclo[3.3.1]nonan-9-one-1,5-dicarboxylate (N2py3o-CI). The preferred TACN-Nx ligand is 1,4,7-trimethyl-1,4,7-triazacyclononane (Me₃-TACN).

The transition metal salt or complex is preferably present in the accelerator solution resulting from the process according to the present invention, determined as metal, in an amount of at least 50 mmol/l, more preferably at least 100 mmol/l. It is preferably present in the accelerator solution in an amount of less than 5000 mmol/l, more preferably less than 2500 mmol/l, and most preferably less than 1000 mmol/l.

Suitable nitrogen-containing bases to be used in the process of the present invention are tertiary amines such as triethyl amine, dimethylaniline, diethylaniline, or N,N-dimethyl-p-toludine (DMPT), polyamines such as 1,2-(dimethyl amine)ethane, secondary amines such as diethyl amine, ethoxylated amines such as triethanol amine, dimethylamino ethanol, diethanol amine, or monoethanol amine, and aromatic amines such as bipyridine.

The nitrogen-containing base is preferably present in the accelerator solution resulting from the process according to the present invention in an amount of 5-50 wt%. In the pre-accelerator resin it is preferably present in an amount of 0.5-10 g/kg resin.

The term "hydroxy-functional solvent" includes compounds of the formula HO-(-CH₂-C(R¹)₂-(CH₂)ₘ-O-)ₙ-R², wherein each R¹ is independently selected from the group consisting of hydrogen, alkyl groups with 1-10 carbon atoms, and hydroxyalkyl groups with 1 to 10 carbon atoms, n=1-10, m=0 or 1, and R² is hydrogen or an alkyl group with 1-10 carbon atoms. Most preferably, each R¹ is independently selected from H, CH₃, and CH₂OH. Examples of suitable hydroxy-functional solvents are glycols like diethylene glycol monobutyl ether, ethylene glycol, diethylene glycol, dipropylene glycol, and polyethylene glycols, glycerol, and pentaerythritol. The hydroxy-functional solvent is preferably present in the accelerator solution resulting from the process according to the present invention in an amount of 1-50 wt%, preferably 5-30 wt%.

The accelerator solution resulting from the process of the present invention may optionally contain one or more promoters, water, reducing agents, additives, and/or fillers.

There are two important classes of promoters: metal carboxylate salts, phosphorous-containing compounds, and 1,3-diketones.

Examples of 1,3-diketones are acetyl acetone, benzoyl acetone, and dibenzoyl methane, and acetoacetates such as diethyl acetoacetamide, dimethyl acetoacetamide, dipropylacetoacetamide, dibutylacetoacetamide, methyl acetoacetate, ethyl acetoacetate, propyl acetoacetate, and butylacetoacetate.

Examples of phosphorous-containing compounds are phosphorous compounds with the formulae P(R)₃ and P(R)₃=O, wherein each R is independently selected from hydrogen, alkyl with 1 to 10 carbon atoms, and alkoxy groups with 1 to 10 carbon atoms. Preferably, at least two R-groups are selected from either alkyl groups of alkoxy groups. Specific examples of suitable phosphorous-containing compounds are diethyl phosphate, dibutyl phosphate, tributyl phosphate, triethyl phosphate (TEP), dibutyl phosphite, and triethyl phosphate.

Examples of suitable metal carboxylate salts are the 2-ethyl hexanoates, octanoates, nonanoates, heptanoates, neodecanoates, and naphthenates of ammonium, alkali metals, and alkaline earth metals. A preferred alkali metal is K. The salts may be added in the process as such, or they may be formed *in situ.* For example, alkali metal 2-ethyl hexanoates can be prepared *in situ,* by addition of an alkali metal hydroxide and 2-ethyl hexanoic acid to a solution. This preparation is exothermic and its reaction heat can be used to heat the solution in the process of the present invention. Therefore, in a preferred embodiment, a first step in the process of the invention involves the preparation of an alkali metal 2-ethyl hexanoate in the hydroxy-functional solvent, prior to the addition of the nitrogen-containing base or any other promoter. The nitrogen-containing base is then added to the resulting warm alkali metal 2-ethyl hexanoate solution, followed by the addition of the transition metal salt.

Acetoacetates are particularly preferred promoters. Particularly preferred is diethyl acetoacetamide. Even more preferred is a combination of diethyl acetoacetamide and potassium 2-ethyl hexanoate. Also preferred is a combination of diethyl acetoacetamide and dibutyl phosphate.

If one or more promoters is/are present in the accelerator solution resulting from the process according to the present invention, their amount preferably is at least 0.01 wt%, more preferably at least 0.1 wt%, even more preferably at least 1 wt%, more preferably at least 10 wt%, and most preferably at least 20 wt%; preferably not more than 90 wt%, more preferably not more than 80 wt%, and most preferably not more than 70 wt%, all based on the total weight of the accelerator solution.

Other promoters, such as 1,3-diketones and phosphorous-containing compounds, are preferably added to the liquid formulation after dissolution of any metal carboxylate, but before addition of the transition metal salt of complex.

The liquid formulation may further comprise additional organic compounds, such as aliphatic hydrocarbon solvents, aromatic hydrocarbon solvents, and solvents that carry an aldehyde, ketone, ether, ester, alcohol, phosphate, or carboxylic acid group. Examples of suitable solvents are aliphatic hydrocarbon solvents such as white spirit and odourless mineral spirit (OMS), aromatic hydrocarbon solvents such naphthenes and mixtures of naphthenes and paraffins, isobutanol; pentanol; 1,2-dioximes, N-methyl pyrrolidinone, N-ethyl pyrrolidinone; dimethyl formamide (DMF); dimethylsulfoxide (DMSO); 2,2,4-trimethylpentanediol diisobutyrate (TxIB); esters such as dibutyl maleate, dibutyl succinate, ethyl acetate, butyl acetate, mono- and diesters of ketoglutaric acid, pyruvates, and esters of ascorbic acid such as ascorbic palmitate; aldehydes; mono- and diesters, more in particular diethyl malonate and succinates; 1,2-diketones, in particular diacetyl and glyoxal; benzyl alcohol, and fatty alcohols.

The liquid formulation may further contain a reducing agent. Examples of reducing agents are ascorbic acid, sodium formaldehyde sulphoxylate (SFS), reducing sugars like glucose and fructose, oxalic acid, phosphines, phosphites, organic or inorganic nitrites, organic or inorganic sulphites, organic or inorganic sulphides, mercaptanes, and aldehydes, and mixtures thereof. Ascorbic acid, which term in this specification includes L-ascorbic acid and D-isoascorbic acid, is the preferred reducing agent.

If a reducing agent is present in the accelerator solution resulting from the process of the present invention, it is preferably present in an amount of more than 0.1 wt%, preferably at least 1 wt%, and most preferably at least 5%. It is preferably present in an amount of less than 30 wt%, more preferably less than 20 wt%, all based on the total weight of the accelerator solution.

The liquid formulation may optionally comprise water. If present, the water content of the accelerator solution according to the present invention is preferably at least 0.01 wt% and more preferably at least 0.1 wt%. The water content is preferably not more than 50 wt%, more preferably not more than 40 wt%, more preferably not more than 20 wt%, even more preferably not more than 10 wt%, and most preferably not more than 5 wt%, all based on the total weight of the accelerator solution.

The accelerator solution resulting from the process of the present invention can be mixed into a curable resin, thereby resulting in a pre-accelerated resin.

Suitable curable resins include alkyd resins, unsaturated polyester (UP) resins, vinyl ester resins, (meth)acrylate resins, polyurethanes, epoxy resins, and mixtures thereof. Preferred resins are (meth)acrylate resins, UP resins and vinyl ester resins. In the context of the present application, the terms "unsaturated polyester resin" and "UP resin" refer to the combination of unsaturated polyester resin and ethylenically unsaturated monomeric compound. The term "(meth)acrylate resin" refers to the combination of acrylate or methacrylate resin and ethylenically unsaturated monomeric compound. UP resins and acrylate resins as defined above are common practice and commercially available. Curing is generally started by either adding the accelerator solution according to the invention and the initiator (peroxide) to the resin, or by adding the peroxide to the pre-accelerated resin. Suitable UP resins to be cured by the process of the present invention are so-called ortho-resins, iso-resins, iso-npg resins, and dicyclopentadiene (DCPD) resins. Examples of such resins are maleic, fumaric, allylic, vinylic, and epoxy-type resins, bisphenol A resins, terephthalic resins, and hybrid resins.

Vinyl ester resins include acrylate resins, based on, e.g. methacrylate, diacrylate, dimethacrylate, and oligomers thereof.

Acrylate resins include acrylates, methacrylates, diacrylates and dimethacrylates, and oligomers thereof.

Examples of ethylenically unsaturated monomeric compounds include styrene and styrene derivatives like α-methyl styrene, vinyl toluene, indene, divinyl benzene, vinyl pyrrolidone, vinyl siloxane, vinyl caprolactam, stilbene, but also diallyl phthalate, dibenzylidene acetone, allyl benzene, methyl methacrylate, methylacrylate, (meth)acrylic acid, diacrylates, dimethacrylates, acrylamides; vinyl acetate, triallyl cyanurate, triallyl isocyanurate, allyl compounds which are used for optical application (such as (di)ethylene glycol diallyl carbonate), chlorostyrene, tert-butyl styrene, tert-butylacrylate, butanediol dimethacrylate and mixtures thereof. Suitable examples of (meth)acrylates reactive diluents are PEG200 di(meth)acrylate, 1,4-butanediol di(meth)acrylate, 1,3-butanediol di(meth)acrylate, 2,3-butanedioldi(meth)acrylate, 1,6-hexanediol di(meth)acrylate and its isomers, diethyleneglycol di(meth)acrylate,triethyleneglycol di(meth)acrylate, glycerol di(meth)acrylate, trimethylolpropane di(meth)acrylate, neopentyl glycol di(meth)acrylate, dipropyleneglycol di(meth)acrylate, tripropyleneglycol di(meth)acrylate, PPG250 di(meth)acrylate, tricyclodecane dimethylol di(meth)acrylate, 1,10-decanediol di(meth)acrylate, tetraethylene glycol di(meth)acrylate, trimethylolpropanetri(meth)acrylate, glycidyl (meth)acrylate, (bis)maleimides, (bis)citraconimides, (bis)itaconimides, and mixtures thereof.

The amount of ethylenically unsaturated monomer in the pre-accelerated resin is preferably at least 0.1 wt%, based on the weight of the resin, more preferably at least 1 wt%, and most preferably at least 5 wt%. The amount of ethylenically unsaturated monomer is preferably not more than 50 wt%, more preferably not more than 40 wt%, and most preferably not more than 35 wt%.

If the accelerator solution resulting from the process according to the present invention is used for curing a resin or for preparing a pre-accelerated resin, the accelerator solution is generally employed in amounts of at least 0.01 wt%, preferably at least 0.1 wt%, and preferably not more than 5 wt%, more preferably not more than 3 wt% of the accelerator solution, based on the weight of the resin.

Peroxides suitable for curing the resin and suitable for being present in the second component of the two-component composition include inorganic peroxides and organic peroxides, such as conventionally used ketone peroxides, peroxyesters, diaryl peroxides, dialkyl peroxides, and peroxydicarbonates, but also peroxycarbonates, peroxyketals, hydroperoxides, diacyl peroxides, and hydrogen peroxide. Preferred peroxides are organic hydroperoxides, ketone peroxides, peroxyesters, and peroxycarbonates. Even more preferred are hydroperoxides and ketone peroxides. Preferred hydroperoxides include cumyl hydroperoxide, 1,1,3,3-tetramethylbutyl hydroperoxide, tert-butyl hydroperoxide, isopropylcumyl hydroperoxide, tert-amyl hydroperoxide, 2,5-dimethylhexyl-2,5-dihydroperoxide, pinane hydroperoxide, and pinene hydroperoxide. Preferred ketone peroxides include methyl ethyl ketone peroxide, methyl isopropyl ketone peroxide, methyl isobutyl ketone peroxide, cyclohexanone peroxide, and acetylacetone peroxide.

Of course, also mixtures of two or more peroxides can be used; for instance a combination of a hydroperoxide or ketone peroxide with a peroxyester.

A particularly preferred peroxide is methyl ethyl ketone peroxide. The skilled person will understand that these peroxides can be combined with conventional additives, for instance fillers, piments, and phlegmatisers. Examples phlegmatizers are hydrophilic esters and hydrocarbon solvents. The amount of peroxide to be used for curing the resin is preferably at least 0.1 per hundred resin (phr), more preferably at least 0.5 phr, and most preferably at least 1 phr. The amount of peroxide is preferably not more than 8 phr, more preferably not more than 5 phr, most preferably not more than 2 phr.

When the peroxide is mixed with the pre-accelerated resin, it is added to a pre-mix of resin and accelerator solution, or is pre-mixed with the resin after which accelerator solution is added. The resulting mixture is mixed and dispersed. The curing process can be carried out at any temperature from -15°C up to 250°C, depending on the initiator system, the accelerator system, the compounds to adapt the curing rate, and the resin composition to be cured. Preferably, it is carried out at ambient temperatures commonly used in applications such as hand lay-up, spray-up, filament winding, resin transfer moulding, coating (e.g. gelcoat and standard coatings), button production, centrifugal casting, corrugated sheets or flat panels, relining systems, kitchen sinks via pouring compounds, etc. However, it can also be used in SMC, BMC, pultrusion techniques, and the like, for which temperatures up to 180°C, more preferably up to 150°C, most preferably up to 100°C, are used.

Other optional additives may be employed in the curing process according to the invention, such as fillers, glass fibres, pigments, inhibitors, and promoters.

The cured resins find use in various applications, including marine applications, chemical anchoring, roofing, construction, relining, pipes and tanks, flooring, windmill blades, etc.

### EXAMPLES

Accelerator solutions were prepared in different ways using the same ingredients: 20 wt% potassium octanoate (K-oct), 25 wt% diethanolamine (DEA), 45 wt% N,N-diethylacetoacetamide (DEAA), 5 wt% copper(II) acetate (CuAc), and 5 wt% diethylene glycol (DEG). The potassium octanoate was made in advance from 2-ethyl hexanoic acid and potassium hydroxide (90%), in said DEG.

In step 1, (part of) the ingredients listed in Table 1 were mixed. Except for Example 1, this was done at 20°C. In Example 1, use was made of the reaction heat evolved when preparing the potassium octanoate.

The solubility of copper in the prepared solutions at 20°C after 4 hours and 24 hours was judged visibly.

Step 2 involved addition of the remaining ingredients and optionally heating the mixture to 85°C, in different orders. The solubility was judged visibly again.

In this Table,
+ means "poor"
++ means "moderate"
+++ means "incomplete"
++++ means "complete"

These accelerator solutions - 1 phr (per hundred resin) solution - were used to cure an ortho phthalic acid-based unsaturated polyester resin (Palatal® P6 ex DSM resin) at 20°C with 2 phr methyl ethyl ketone peroxide (Butanox® M50, ex-AkzoNobel).

The curing performance was analysed by the method of the Society of Plastic Institute (SPI method F/77.1; available from Akzo Nobel Polymer Chemicals). This method involves measuring the peak exotherm, the time to peak, and the gel time. According to this method, 25 g of a mixture comprising 100 parts of resin , 2 parts of peroxide, and 1 part of accelerator solution were poured into a test tube and a thermocouple was placed through the enclosure at the centre of the tube. The glass tube was then placed in a climate controlled room maintained at 20°C and the time-temperature curve was measured. From the curve the following parameters were calculated:
Gel time (Gt) = time in minutes elapsed between the start of the experiment and 5.6°C above the bath temperature.
Time to peak (TTP) = time elapsed between the start of the experiment and the moment that the peak temperature is reached.
Peak exotherm (PE) = the maximum temperature that is reached.

The results are displayed in Table 1.

**Table 1**

| Example: | 1 | 2a (comp) | 2b (comp) | 3 (comp) | 4 (comp) | 5 (comp) | 6 (comp) |
|---|---|---|---|---|---|---|---|
| **Step 1** | K-Oct | K-Oct | K-Oct | K-Oct | K-Oct | K-Oct | DEA |
| | | DEA | DEA | CuAc | DEA | DEAA | DEAA |
| | | DEAA | DEAA | | CuAc | CuAc | CuAc |
| | | CuAc | CuAc | | | | |
| Temp: | 85°C | 20°C | 20°C | 20°C | 20°C | 20°C | 20°C |
| Solubility | | | | | | | |
| 4 hrs | | +++ | +++ | + | ++ | + | ++ |
| 24 hrs | | ++++ | ++++ | ++ | +++ | ++ | ++++ |
| **Step 2** | | | | | | | |
| Heating step | still 85°C | still 20°C | | still 20°C | to 85°C (30 min) | to 85°C (30 min) | to 85°C (30 min) |
| Addition | DEA | - | - | DEA | DEAA | DEA | K-Oct |
| | DEAA | | | DEAA | | | |
| | CuAc | | | | | | |
| heating step | - still 85°C | - still 20°C | to 85°C (30 min) | to 85°C (30 min) | still 85°C | still 85°C | - still 85°C |
| **Solubility** | ++++ | ++++ | ++++ | ++++ | ++++ | ++++ | ++++ |
| **Gt (min)** | 11 | 16 | 15 | 13 | 14 | 13 | 14 |
| **TTP (min)** | 14 | 20 | 19 | 17 | 17 | 16 | 17 |
| **PE (°C)** | 169 | 163 | 168 | 171 | 169 | 171 | 170 |
| **Preparation time (hrs)** | 0.5 | >24 | >4 | >24 | >4 | >24 | >4 |

These results show that the order of addition and heating steps affect the required preparation time (mainly because of a different dissolution speed) and the performance of the accelerator solution. The effect on performance may look insignificant at first sight, but it is not. Gel time differences of 2 minutes are significant in daily practice.

## Claims

1. Process for the preparation of an accelerator solution suitable for forming a redox system with peroxides, comprising the step of adding a transition metal salt or complex to a liquid formulation comprising a hydroxy-functional solvent and a nitrogen-containing base at a temperature in the range 50-200°C.

2. Process according to claim 1 wherein the transition metal is selected from the group consisting of Cu, Mn, Fe, and V.

3. Process according to claim 1 or 2 wherein the liquid formulation comprises an alkali or alkaline earth metal compound, said alkali or alkaline earth metal compound being dissolved in the hydroxy-functional solvent prior to introduction of the nitrogen-containing base.

4. Accelerator solution suitable for forming a redox system with peroxides obtainable by the process of any one of claims 1-3.

5. Process for pre-accelerating a curable resin, comprising the step of mixing the accelerator solution of claim 4 into the curable resin.

6. Pre-accelerated unsaturated polyester resin or vinyl ester resin obtainable according to the process of claim 5.

7. Two-component composition comprising a first component and a second component, the first component comprising the pre-accelerated resin composition according to claim 6, the second component comprising a peroxide.

8. Two component composition according to claim 7 wherein the peroxide is selected from the group consisting of organic hydroperoxides, ketone peroxides, peroxycarbonates, and peroxyesters.

## Patentansprüche

1. Verfahren zur Herstellung einer Beschleunigerlösung, die zur Bildung eines Redoxsystems mit Peroxiden geeignet ist, umfassend den Schritt des Zufügens eines Übergangsmetallsalzes oder -komplexes zu einer Flüssigformulierung, umfassend ein hydroxy-funktionelles Lösungsmittel und eine stickstoffhaltige Base bei einer Temperatur in dem Bereich von 50-200°C.

2. Verfahren nach Anspruch 1, wobei das Übergangsmetall aus der Gruppe ausgewählt ist, die Cu, Mn, Fe und V aufweist.

3. Verfahren nach Anspruch 1 oder 2, wobei die Flüssigformulierung eine Alkali- oder eine Erdalkalimetallverbindung umfasst, wobei die Alkali- oder die Erdalkalimetallverbindung vor der Zuführung der stickstoffhaltigen Base in dem hydroxy-funktionellen Lösungsmittel aufgelöst wird.

4. Beschleunigerlösung, die für die Bildung eines Redoxsystems mit Peroxiden geeignet ist, welche durch das Verfahren nach einem der Ansprüche 1-3 erhältlich ist.

5. Verfahren zur Vorbeschleunigung eines härtbaren Harzes, umfassend den Schritt des Mischens der Beschleunigerlösung von Anspruch 4 mit dem härtbaren Harz.

6. Vorbeschleunigtes, ungesättigtes Polyesterharz oder Vinylesterharz, das nach dem Verfahren von Anspruch 5 erhältlich ist.

7. Zweikomponenten-Zusammensetzung umfassend eine erste Komponente und eine zweite Komponente, wobei die erste Komponente die vorbeschleunigte Harzzusammensetzung nach Anspruch 6 umfasst, und die zweite Komponente ein Peroxid umfasst.

8. Zweikomponenten-Zusammensetzung nach Anspruch 7, wobei das Peroxid aus der Gruppe ausgewählt ist, die organische Hydroperoxide, Ketonperoxide, Peroxycarbonate und Peroxyester aufweist.

## Revendications

1. Procédé pour la préparation d'une solution accélératrice appropriée pour la formation d'un système redox avec des peroxydes, comprenant l'étape d'ajout d'un sel ou complexe de métal de transition à une formulation liquide comprenant un solvant à fonction hydroxy et une base contenant de l'azote à une température se trouvant dans la plage allant de 50 à 200°C.

2. Procédé selon la revendication 1, dans lequel le métal de transition est choisi dans le groupe constitué de Cu, Mn, Fe, et V.

3. Procédé selon la revendication 1 ou 2, dans lequel la formulation liquide comprend un composé de métal alcalin ou alcalino-terreux, ledit composé de métal alcalin ou alcalino-terreux étant dissous dans le solvant à fonction hydroxy avant l'introduction de la base contenant de l'azote.

4. Solution accélératrice appropriée pour la formation d'un système redox avec des peroxydes pouvant être obtenue par le procédé de l'une quelconque des revendications 1 à 3.

5. Procédé pour la pré-accélération d'une résine durcissable, comprenant l'étape de mélange de la solution accélératrice de la revendication 4 dans la résine durcissable.

6. Résine de polyester insaturé ou résine d'ester vinylique pré-accélérée pouvant être obtenue selon le procédé de la revendication 5.

7. Composition à deux composants comprenant un premier composant et un deuxième composant, le premier composant comprenant la composition de résine pré-accélérée selon la revendication 6, le deuxième composant comprenant un peroxyde.

8. Composition à deux composants selon la revendication 7, dans laquelle le peroxyde est choisi dans le groupe constitué d'hydroperoxydes organiques, de peroxydes de cétone, de peroxycarbonates, et de peroxyesters.
